# EUROPEAN PATENT SPECIFICATION

(11) **EP 3 328 349 B1**
(45) Date of publication and mention of the grant of the patent: **25.12.2019**
(21) Application number: 16753840.4
(22) Date of filing: 28.07.2016
(51) Int. Cl.: A61K 8/26, A61K 8/31, A61Q 15/00, A61K 8/81, A61K 8/89, A61K 8/891, A61K 8/04, A61K 8/28

(54) **ANHYDROUS COMPOSITION IN AEROSOL FORM COMPRISING AN ANTIPERSPIRANT ACTIVE AGENT AND A DISPERSION OF POLYMER PARTICLES IN A NON-AQUEOUS MEDIUM**
WASSERFREIE ZUSAMMENSETZUNG IN AEROSOLFORM MIT EINEM SCHWEISSHEMMENDEN WIRKSTOFF UND DISPERSION AUS POLYMERPARTIKELN IN EINEM WASSERFREIEN MEDIUM
ANHYDROUS COMPOSITION IN AEROSOL FORM COMPRISING AN ANTIPERSPIRANT ACTIVE AGENT AND A DISPERSION OF POLYMER PARTICLES IN A NON-AQUEOUS MEDIUM

(30) Priority: 28.07.2015 FR 1557189
(43) Date of publication of application: 06.06.2018
(73) Proprietor: L'OREAL, 75008 Paris (FR)
(72) Inventor: JALENQUES, Xavier, 94152 Chevilly La Rue (FR); RAMOS-STANBURY, Laure, 92160 Antony (FR)
(74) Representative: L'Oreal
(86) International application number: PCT/EP2016/068080
(87) International publication number: WO 2017/017222

(56) References cited:
- FR-A1- 2 937 645
- FR-A1- 2 998 782
- FR-A1- 3 014 875
- FR-A1- 3 015 260

## Description

The present invention relates to an anhydrous composition in aerosol form comprising, in particular in a physiologically acceptable medium:
a) an oily phase comprising at least one volatile oil A, and
b) at least one antiperspirant active agent chosen from aluminium and/or zirconium salts or complexes, and
c) at least one particular dispersion of polymer particles in a non-aqueous medium that will be defined later in detail, and
d) at least one propellant.

The invention also relates to a cosmetic process for treating human perspiration, and optionally the body odours associated with human perspiration, in particular underarm odours, comprising the application of said composition to a surface of the skin.

The present invention also relates to the use of said cosmetic composition and also to an aerosol device using it.

The armpits and also certain other parts of the body are generally the site of much discomfort that may arise directly or indirectly from perspiration. This perspiration often leads to unpleasant and disagreeable sensations that are mainly due to the presence of sweat resulting from perspiration, which may, in certain cases, make the skin and clothing wet, in particular in the region of the armpits or of the back, thus leaving visible marks. Finally, during its evaporation, sweat may also leave salts and/or proteins on the surface of the skin, which thus results in whitish marks on clothing. Such discomfort is noticed, including in the case of moderate perspiration.
In the cosmetics field, it is thus well known to use, in topical application, antiperspirant products containing substances that have the effect of limiting or even preventing the flow of sweat in order to overcome the problems mentioned above. These products are generally available in the form of rollons, sticks, aerosols or sprays.

Antiperspirant substances are generally constituted of aluminium and/or zirconium salts or complexes, such as aluminium chloride and aluminium hydroxyhalides. These substances make it possible to reduce the flow of sweat.

However, certain cosmetic compositions based on these antiperspirant substances generally have a tendency to become transferred onto clothing, leaving unsightly, visible marks.

In order to overcome the problem of marks, antiperspirant compositions containing oils with a refractive index close to those of aluminium salts have been developed. The role of such oils is to reduce the whitish appearance of antiperspirant compositions when they are deposited on the skin and, consequently, to make the marks on clothing less white. The oils used are generally fatty acid esters such as isopropyl myristate or polydimethylsiloxanes. Such compositions have been particularly described in patents or patent applications EP 1362885, JP 03095111 and FR 2242969.

However, such antiperspirant compositions have the drawback of giving the skin, in particular on the armpits, an oily sensation that the user finds unpleasant, and do not make it possible to limit the transfer of antiperspirant products from the skin onto clothing.

There is thus a real need to use antiperspirant cosmetic compositions in aerosol form, which do not have the drawbacks mentioned above, i.e. compositions which make it possible to achieve less transfer onto fabrics, while maintaining good antiperspirant efficacy.

Patent application FR3015260 discloses an anhydrous antiperspirant composition in aerosol form comprising a water-insoluble film-forming block ethylenic polymers, which becomes transferred as little as possible onto fabrics and which maintain antiperspirant efficacy.

The applicant has therefore discovered, surprisingly, that, by applying to the skin an anhydrous composition in aerosol form comprising, in particular in a physiologically acceptable medium:
a) an oily phase comprising at least one volatile oil A, and
b) at least one antiperspirant active agent chosen from aluminium and/or zirconium salts or complexes, and
c) at least one particular dispersion of polymer particles in a non-aqueous medium that will be defined later in detail, and
d) at least one propellant.

Thus, the antiperspirant cosmetic composition in aerosol form in accordance with the invention makes it possible to produce formulae which transfer less onto textiles, thus giving rise to fewer unsightly visible marks on clothing, in particular on dark-coloured clothing, when compared with a standard antiperspirant composition or an antiperspirant composition containing oils and to maintain good antiperspirant efficacy.

In particular, the antiperspirant cosmetic composition makes it possible to significantly reduce the whitish marks on clothing, in particular on dark-coloured clothing.

Moreover, the antiperspirant cosmetic composition in aerosol form in accordance with the invention can lead to formulae which transfer less onto textiles, thus giving rise to fewer unsightly visible marks on clothing, in particular on dark-coloured clothing, when compared with a standard antiperspirant composition or an antiperspirant composition containing oils.

Thus, the use of this particular dispersion of polymer particles in a non-aqueous medium makes it possible to reduce the transfer of unsightly visible marks on the clothing without harming the efficacy of the aluminium salts. Furthermore, this dispersion proves to be compatible with the aluminium and/or zirconium salts or complexes since they do not form a macroscopically visible precipitate in the composition. This same dispersion also proves to be compatible with propellant gases.

A subject of the present invention is therefore an anhydrous composition in aerosol form comprising, in particular in a physiologically acceptable medium:
a) an oily phase comprising at least one volatile oil A, and
b) at least one antiperspirant active agent chosen from aluminium and/or zirconium salts or complexes, and
c) at least one particular dispersion of polymer particles in a non-aqueous medium that will be defined later in detail, and
d) at least one propellant.

The cosmetic composition according to the invention has both good transfer-resistance and antiperspirant properties.

Moreover, the present invention also relates to a cosmetic process for treating human perspiration, and optionally body odours associated with human perspiration, which consists in applying to the surface of a human keratin material a cosmetic composition as described previously.

The process according to the invention is particularly advantageous for treating armpit perspiration, since the composition used does not give an unpleasant oily sensation and transfers less onto clothing, while at the same time efficiently treating perspiration.

The invention also relates to the use of said composition for cosmetically treating human perspiration.

Other subjects, characteristics, aspects and advantages of the invention will become even more clearly apparent on reading the description and the examples which follow.

For the purposes of the present invention, the term "physiologically acceptable medium" is intended to denote a medium that is suitable for the topical administration of a composition, i.e. a medium which is colourless and has no unpleasant appearance, and which is perfectly compatible with the topical route of administration. In the present case, where the composition is intended for topical administration, i.e. by application at the surface of the keratin material under consideration, such a medium is considered in particular to be physiologically acceptable when it does not cause stinging, tautness or redness that is unacceptable to the user.

For the purposes of the present invention, the term "anhydrous" is intended to mean a liquid phase with a water content of less than 5% by weight, preferably less than 2% by weight and even more preferably less than 1% by weight relative to the weight of said composition. It should be noted that the water in question is more particularly bound water, such as the water of crystallization in salts, or traces of water absorbed by the raw materials used in the production of the compositions according to the invention.

The term "human keratin materials" is intended to mean the skin (of the body, face and around the eyes), hair, eyelashes, eyebrows, body hair, nails, lips or mucous membranes.

The term "final composition" is intended to mean the combination of the liquid phase and of the propellant gas.

### Antiperspirant active agent

The term "antiperspirant active agent" is intended to mean a salt which, by itself, has the effect of reducing the flow of sweat, of reducing the sensation on the skin of moisture associated with human sweat and of masking human sweat.

As indicated previously, the cosmetic composition comprises one or more antiperspirant active agents chosen from aluminium and/or zirconium salts or complexes.

Among the aluminium salts or complexes, mention may be made of aluminium halohydrates.

Among the aluminium salts, mention may in particular be made of aluminium chlorohydrate, aluminium chlorohydrex, the aluminium chlorohydrex-polyethylene glycol complex, the aluminium chlorohydrex-propylene glycol complex, aluminium dichlorohydrate, the aluminium dichlorohydrex-polyethylene glycol complex, the aluminium dichlorohydrex-propylene glycol complex, aluminium sesquichlorohydrate, the aluminium sesquichlorohydrex-polyethylene glycol complex, the aluminium sesquichlorohydrex-propylene glycol complex, aluminium sulfate buffered with sodium aluminium lactate. Aluminium sesquichlorohydrate is in particular sold under the trade name Reach 301® by the company Summitreheis.

Aluminium chlorohydrate is in particular sold under the trade names Locron S FLA®, Locron P and Locron L.ZA by the company Clariant; under the trade names Microdry Aluminium Chlorohydrate®, Micro-Dry 323®, Chlorhydrol 50, Reach 103 and Reach 501 by the company Summitreheis; under the trade name Westchlor 200® by the company Westwood; under the trade name Aloxicoll PF 40® by the company Guilini Chemie; Cluron 50%® by the company Industria Quimica Del Centro; or Clorohidroxido Aluminio SO A 50%® by the company Finquimica.

Aluminium chlorohydrate, aluminium sesquichlorohydrate and mixtures thereof will more particularly be used.

The aluminium and/or zirconium salts or complexes may be present in the final composition according to the invention in a content ranging from 1% to 25% by weight, preferably in a content ranging from 2% to 20% and more particularly between 3% and 15% by weight relative to the total weight of the final composition.

### Dispersion of polymer particles

The dispersion of polymer particles in accordance with the invention is a dispersion of particles of methyl acrylate/ethyl acrylate (50/50 by weight) copolymer, stabilized by a random copolymer stabilizer containing 75.2% by weight of isobornyl acrylate, 3% of methyl acrylate, 3% of ethyl acrylate and 18.8% of monomethacryloyloxypropyl polydimethylsiloxane, in isododecane.

The presence of the silicone macromonomer in the stabilizer and/or the polymer of the particles makes it possible to obtain a polymer dispersion that is stable, in particular after storage for 7 days at ambient temperature (25°C).

The dispersions according to the invention are thus constituted of particles, which are generally spherical, of at least one polymer in a non-aqueous medium.

The polymer of the particles may be present in the dispersion in a content ranging from 20% to 60% by weight, relative to the total weight of the dispersion.

The stabilizer is in contact with the surface of the polymer particles and thus makes it possible to stabilize these particles at the surface in order to keep these particles in dispersion in the non-aqueous medium of the dispersion. Thus, the polymer particles are surface-stabilized by the stabilizer. The stabilizer is a polymer distinct from the polymer of the particles: the stabilizer does not form a covalent bond with the polymer of the particles.

The polymer particles of the dispersion preferably have an average size, in particular a number-average size, ranging from 50 to 500 nm, in particular ranging from 75 to 400 nm and better still ranging from 100 to 250 nm.

### Non-aqueous medium of the dispersion

The non-aqueous medium of the polymer dispersion is isododecane.

### Preparation of the dispersion of polymer particles

In general, the dispersion according to the invention may be prepared in the following manner, which is given as an example.

The polymerization may be performed in dispersion, i.e. by precipitation of the polymer during formation, with protection of the formed particles with a stabilizer.

In a first step, the stabilizing polymer is prepared by mixing the constituent monomer(s) of the stabilizing polymer, with a radical initiator, in a solvent known as the synthesis solvent, and by polymerizing these monomers. In a second step, the constituent monomer(s) of the polymer of the particles are added to the stabilizing polymer formed and polymerization of these added monomers is performed in the presence of the radical initiator.

The polymerization may be performed directly in isododecane which thus also acts as synthesis solvent. The monomers should also be soluble therein, as should the radical initiator, and the polymer of the particles obtained should be insoluble therein.

The monomers are preferably present in the synthesis solvent, before polymerization, in a proportion of 5-20% by weight. The total amount of the monomers may be present in the solvent before the start of the reaction, or part of the monomers may be added gradually as the polymerization reaction proceeds.

The radical initiator may in particular be azobisisobutyronitrile or tert-butyl peroxy-2-ethylhexanoate.

The polymerization may be performed at a temperature ranging from 70 to 110°C.

The polymer particles are surface-stabilized, when they are formed during the polymerization, by means of the stabilizer.

The stabilization may be performed by any known means, and in particular by direct addition of the stabilizer, during the polymerization.

The stabilizer is preferably also present in the mixture before polymerization of the monomers of the polymer of the particles. However, it is also possible to add it continuously, in particular when the monomers of the polymer of the particles are also added continuously.

### Oily phase of the composition

The antiperspirant composition according to the invention comprises an oily phase comprising at least one volatile oil A.

For the purposes of the invention, the term "volatile oil" is intended to mean an oil that is capable of evaporating on contact with the skin or the keratin fibre in less than one hour, at ambient temperature and atmospheric pressure.

The total amount of oil(s) present in the composition of the invention is preferably in a content ranging from 10% to 90% by weight and more preferentially in a content ranging from 15% to 80% by weight, even more preferentially in a content ranging from 20% to 60% by weight, relative to the total weight of the liquid phase (or of the fluid).

For the purposes of the present invention, the term "liquid phase" or "fluid" is intended to mean the base of the composition without the propellant.

The volatile oils A of the invention are volatile cosmetic oils that are liquid at ambient temperature with a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

Preferentially, the volatile oil A is chosen from hydrocarbon-based volatile oils and silicone volatile oils, or mixtures thereof.

The hydrocarbon-based volatile oil A that may be used in the invention is chosen from the volatile hydrocarbon-based oils having from 8 to 16 carbon atoms.

Among the volatile hydrocarbon-based oils having from 8 to 16 carbon atoms, mention may be made of:
- branched C8 -C16 alkanes, for instance C8 -C16 isoalkanes of petroleum origin (also known as isoparaffins), for instance isododecane (also known as 2,2,4,4,6-pentamethylheptane), isodecane, isohexadecane and, for example, the oils sold under the trade name Isopar or Permethyl,
- linear alkanes, for instance n-dodecane (C12) and n-tetradecane (C14) sold by Sasol under the respective references Parafol 12-97 and Parafol 14-97, and also mixtures thereof, the undecane-tridecane mixture, the mixtures of n-undecane (C11) and of n-tridecane (C13) obtained in Examples 1 and 2 of application WO 2008/155 059 from the company Cognis, and mixtures thereof.

As examples of volatile silicone oils A that may be used in the invention, mention may be made of:
- volatile linear or cyclic silicone oils, in particular those with a viscosity ≤ 8 centistokes (8×10⁻⁶ m²/s) and in particular containing from 2 to 7 silicon atoms, these silicones optionally comprising alkyl or alkoxy groups having from 1 to 10 carbon atoms. As volatile silicone oils that may be used in the invention, mention may be made in particular of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane;
- volatile linear alkyltrisiloxane oils of general formula (II): 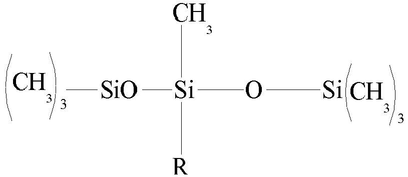 in which R represents an alkyl group comprising from 2 to 4 carbon atoms, one or more hydrogen atoms of which may be replaced with a fluorine or chlorine atom.

Among the oils of general formula (II), mention may be made of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (II) for which R is, respectively, a butyl group, a propyl group or an ethyl group.

The proportion of volatile oil(s) A relative to the total amount of the oils preferably ranges from 50% to 100% by weight.

Preferably, the volatile oils A are chosen from hydrocarbon-based oils and more particularly C₈-C₁₆ isoalkanes such as isododecane or isohexadecane, or linear C₈-C₁₆ alkanes such as an undecane/tridecane mixture.

Even more particularly, isododecane will be chosen.

The oily phase of the composition may also comprise one or more non-volatile oils chosen from non-volatile hydrocarbon-based oils and non-volatile silicone oils.

The non-volatile hydrocarbon-based oils that may be used in the invention may be chosen from the non-volatile hydrocarbon-based oils having from 8 to 16 carbon atoms.

Among the non-volatile hydrocarbon-based oils having from 8 to 16 carbon atoms, mention may be made of:
- short-chain esters (containing from 3 to 8 carbon atoms in total) such as ethyl acetate, methyl acetate, propyl acetate or n-butyl acetate;
- hydrocarbon-based oils of plant origin such as triglycerides constituted by fatty acid esters of glycerol, the fatty acids of which may have chain lengths varying from C4 to C24 , these chains possibly being linear or branched, and saturated or unsaturated; these oils are in particular heptanoic or octanoic acid triglycerides, or alternatively wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion-flower oil and musk rose oil; shea butter; or else caprylic/capric acid triglycerides, for instance those sold by the company Stearineries Dubois or those sold under the names Miglyol 810®, 812® and 818® by the company Dynamit Nobel;
- synthetic ethers having from 10 to 40 carbon atoms;
- linear or branched hydrocarbons of mineral or synthetic origin, such as petroleum jelly, polydecenes, hydrogenated polyisobutene such as Parleam®, squalane and liquid paraffins, and mixtures thereof;
- synthetic esters such as oils of formula R1 COOR2 in which R1 represents a linear or branched fatty acid residue containing from 1 to 40 carbon atoms and R2 represents an in particular branched hydrocarbon-based chain containing from 1 to 40 carbon atoms, on the condition that R1 + R2 ≥ 10, for instance purcellin oil (cetostearyl octanoate), isopropyl myristate, isopropyl palmitate, C12 to C15 alkyl benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, alkyl or polyalkyl heptanoates, octanoates, decanoates or ricinoleates such as propylene glycol dioctanoate; hydroxylated esters such as isostearyl lactate, diisostearyl malate and 2-octyldodecyl lactate; polyol esters and pentaerythritol esters;
- fatty alcohols that are liquid at ambient temperature, with a branched and/or unsaturated carbon-based chain having from 12 to 26 carbon atoms, for instance octyldodecanol, isostearyl alcohol, oleyl alcohol, 2-hexyldecanol, 2-butyloctanol and 2-undecylpentadecanol.

As examples of additional non-volatile silicone oils that may be used in the invention, mention may be made of:
- silicone oils, for instance non-volatile polydimethylsiloxanes (PDMSs); polydimethylsiloxanes comprising alkyl, alkoxy or phenyl groups, which are pendant or at the end of a silicone chain, these groups containing from 2 to 24 carbon atoms; phenyl trimethicones, phenyl dimethicones, phenyltrimethylsiloxydiphenylsiloxanes, diphenyl dimethicones, diphenylmethyldiphenyltrisiloxanes, and mixtures thereof.

According to one particular form of the invention, the amount of non-volatile polydimethylsiloxane(s) will be at most 10% by weight, relative to the total weight of the oils.

Preferably, the non-volatile hydrocarbon-based oils will be chosen from hydrogenated polyisobutene oils such as Parleam®, ethers such as dicaprylyl ether or PPG-14 butyl ether, fatty acid esters such as isopropyl palmitate, isononyl isononanoate or C₁₂-C₁₅ alkyl benzoates, fatty alcohols such as octyldodecanol, and mixtures thereof.

Fatty acid esters such as isopropyl palmitate, isononyl isononanoate or C₁₂-C₁₅ alkyl benzoates, and even more particularly isopropyl palmitate, will be chosen more preferentially.

The non-volatile hydrocarbon-based oil(s) may be present in the liquid phase of the composition in a content ranging from 0% to 50% by weight, relative to the total oils of the composition.

### ADDITIVES

The cosmetic compositions according to the invention may also comprise cosmetic adjuvants chosen from deodorant active agents, moisture absorbers, lipophilic suspension agents or gelling agents, softeners, antioxidants, opacifiers, stabilizers, moisturizers, vitamins, bactericides, preserving agents, polymers, fragrances, thickeners or suspension agents or any other ingredient usually used in cosmetics for this type of application.

Needless to say, those skilled in the art will take care to choose this or these optional additional compounds such that the advantageous properties intrinsically associated with the cosmetic composition according to the invention are not, or are not substantially, adversely affected by the envisaged addition(s).

### Deodorant active agents

According to a particular form of the invention, the compositions may contain at least one deodorant active agent in the liquid phase.

The expression "deodorant active agent" is intended to mean any substance capable of reducing, masking or absorbing human body odours, in particular underarm odours.

The deodorant active agents may be bacteriostatic agents or bactericides that act on underarm odour microorganisms, such as 2,4,4'-trichloro-2'-hydroxydiphenyl ether (®Triclosan), 2,4-dichloro-2'-hydroxydiphenyl ether, 3',4',5'-trichlorosalicylanilide, 1-(3',4'-dichlorophenyl)-3-(4'-chlorophenyl)urea (®Triclocarban) or 3,7,11-trimethyldodeca-2,5,10-trienol (®Farnesol); quaternary ammonium salts such as cetyltrimethylammonium salts, cetylpyridinium salts, DPTA (1,3-diaminopropanetetraacetic acid), 1,2-decanediol (Symclariol from the company Symrise); glycerol derivatives, for instance caprylic/capric glycerides (Capmul MCM® from Abitec), glyceryl caprylate or caprate (Dermosoft GMCY® and Dermosoft GMC® from Straetmans), polyglyceryl-2 caprate (Dermosoft DGMC® from Straetmans), and biguanide derivatives, for instance polyhexamethylene biguanide salts; chlorhexidine and salts thereof; 4-phenyl-4,4-dimethyl-2-butanol (Symdeo MPP® from Symrise); zinc salts such as zinc salicylate, zinc gluconate, zinc pidolate, zinc sulfate, zinc chloride, zinc lactate or zinc phenolsulfonate; salicylic acid and derivatives thereof such as 5-n-octanoylsalicylic acid.

The deodorant active agents may be odour absorbers such as zinc ricinoleates or sodium bicarbonate; metallic or silver or silver-free zeolites, or cyclodextrins and derivatives thereof. They may also be chelating agents such as Dissolvine GL-47-S® from Akzo Nobel, EDTA and DPTA. They may also be a polyol such as glycerol or 1,3-propanediol (Zemea Propanediol sold by Dupont Tate and Lyle BioProducts); or also an enzyme inhibitor such as triethyl citrate; or alum.

The deodorant active agents may also be bacteriastatic agents or bactericides 2,4,4'-trichloro-2'-hydroxydiphenyl ether (Triclosan®), 2,4-dichloro-2'-hydroxydiphenyl ether, 3',4',5'-trichlorosalicylanilide, 1-(3',4'-dichlorophenyl)-3-(4'-chlorophenyl)urea (Triclocarban®) or 3,7,11-trimethyldodeca-2,5,10-trienol (Farnesol®); quaternary ammonium salts such as cetyltrimethylammonium salts or cetylpyridinium salts.

The deodorant active agents may be present in the composition according to the invention in a proportion from about 0.01% to 20% by weight relative to the total composition, and preferably in a proportion of from about 0.1% to 5% by weight relative to the total weight of the final composition.

### Moisture absorbers

It is also possible to add moisture absorbers, for instance perlites and preferably expanded perlites.

The cosmetic composition may comprise one or more moisture absorbers chosen from perlites.

Preferably, the cosmetic composition comprises one or more absorbers chosen from expanded perlites.

The perlites that may be used according to the invention are generally aluminosilicates of volcanic origin and have the composition:
70.0-75.0% by weight of silica SiO₂
12.0-15.0% by weight of aluminium oxide Al₂O₃
3.0-5.0% of sodium oxide Na₂O
3.0-5.0% of potassium oxide K₂O
0.5-2% of iron oxide Fe₂O₃
0.2-0.7% of magnesium oxide MgO
0.5-1.5% of calcium oxide CaO
0.05-0.15% of titanium oxide TiO₂

The perlite is ground, dried and then sized in a first step. The product obtained, known as perlite ore, is grey-coloured and has a size of the order of 100 µm.

The perlite ore is subsequently expanded (1000°C/2 seconds) to give relatively white particles. When the temperature reaches 850-900°C, the water trapped in the structure of the material evaporates and brings about the expansion of the material, with respect to its original volume. The expanded perlite particles in accordance with the invention may be obtained via the expansion process described in patent US 5 002 698.

Preferably, the perlite particles used will be ground; in this case, they are known as Expanded Milled Perlite (EMP). They preferably have a particle size defined by a median diameter D50 ranging from 0.5 to 50 µm and preferably from 0.5 to 40 µm.

Preferably, the perlite particles used have an untamped apparent density at 25°C ranging from 10 to 400 kg/m³ (standard DIN 53468) and preferably from 10 to 300 kg/m³.

Preferably, the expanded perlite particles according to the invention have a water absorption capacity, measured at the wet point, ranging from 200% to 1500% and preferably from 250% to 800%.

The wet point corresponds to the amount of water which has to be added to 1 g of particle in order to obtain a homogeneous paste. This method derives directly from the oil uptake method applied to solvents. The measurements are taken in the same manner by means of the wet point and the flow point, which have, respectively, the following definitions:
wet point: mass expressed in grams per 100 g of product corresponding to the production of a homogeneous paste during the addition of a solvent to a powder;
flow point: mass expressed in grams per 100 g of product above which the amount of solvent is greater than the capacity of the powder to retain it. This is reflected by the production of a more or less homogeneous mixture which flows over the glass plate.

The wet point and the flow point are measured according to the following protocol:

### Protocol for measuring the water absorption

### 1) Equipment used

Glass plate (25 x 25 mm)
Spatula (wooden shaft and metal part, 15 x 2.7 mm)
Silk-bristled brush
Balance

### 2) Procedure

The glass plate is placed on the balance and 1 g of perlite particles is weighed out. The beaker containing the solvent and the liquid sampling pipette is placed on the balance. The solvent is gradually added to the powder, the whole being regularly blended (every 3 to 4 drops) with the spatula.

The weight of solvent needed to obtain the wet point is noted. Further solvent is added and the weight which makes it possible to reach the flow point is noted. The average of three tests will be determined.

The expanded perlite particles sold under the trade names Optimat 1430 OR or Optimat 2550 by the company World Minerals will be used in particular.

### Suspension agents/gelling agents

The antiperspirant composition according to the invention may also contain one or more suspension agents and/or one or more gelling agents. Some of them may perform both functions simultaneously.

Among the agents that may be used as lipophilic suspension agents and/or gelling agents, mention may be made of clays, in powder form or in oily gel form, said clays possibly being modified, in particular modified montmorillonite clays such as hydrophobic-modified bentonites or hectorites, for instance hectorites modified with a C₁₀ to C₂₂ ammonium chloride, for instance hectorite modified with distearyldimethylammonium chloride, for instance the product disteardimonium hectorite (CTFA name) (product of reaction of hectorite and of distearyldimonium chloride) sold under the name Bentone 38 or Bentone Gel by the company Elementis Specialities. Mention may be made, for example, of the product Stearalkonium Bentonite (CTFA name) (product of reaction of bentonite and of quaternary stearalkonium ammonium chloride) such as the commercial product sold under the name Tixogel MP 250® by the company Sud Chemie Rheologicals, United Catalysts Inc.

Use may also be made of hydrotalcites, in particular hydrophobic-modified hydrotalcites, for instance the products sold under the name Gilugel by the company BK Giulini.

Mention may also be made of fumed silica optionally hydrophobically treated at the surface, the size of the particles of which is less than 1 µm. It is in fact possible to chemically modify the surface of the silica by chemical reaction which results in a decrease in the number of silanol groups present at the surface of the silica. Silanol groups can in particular be replaced by hydrophobic groups: a hydrophobic silica is then obtained. The hydrophobic groups can be trimethylsiloxyl groups, which are obtained in particular by treatment of fumed silica in the presence of hexamethyldisilazane. Silicas thus treated are named "silica silylate" according to the CTFA (8th edition, 2000). They are sold, for example, under the references Aerosil R812® by the company Degussa, Cab-O-Sil TS-530® by the company Cabot, dimethylsilyloxyl or polydimethylsiloxane groups, which are obtained in particular by treating fumed silica in the presence of polydimethylsiloxane or dimethyldichlorosilane. Silicas thus treated are known as "silica dimethyl silylate" according to the CTFA (8th edition, 2000). They are sold, for example, under the references Aerosil R972® and Aerosil R974® by the company Degussa, and Cab-O-Sil TS-610® and Cab-O-Sil TS-720® by the company Cabot.

The hydrophobic fumed silica in particular has a particle size that may be nanometric to micrometric, for example ranging from about 5 to 200 nm.

According to a particular form of the invention, the suspension agents or gelling agents may be activated with oils such as propylene carbonate or triethyl citrate.

The amounts of these various constituents that may be present in the composition according to the invention are those conventionally used in compositions for treating perspiration.

### Propellant

As indicated previously, the cosmetic composition comprises one or more propellants.

The propellant used in the antiperspirant cosmetic composition according to the invention is chosen from dimethyl ether, volatile hydrocarbons such as propane, isopropane, n-butane, isobutane, n-pentane and isopentane, and mixtures thereof, optionally with at least one chlorohydrocarbon and/or fluorohydrocarbon; among the latter, mention may be made of the compounds sold by the company DuPont de Nemours under the names Freon® and Dymel®, and in particular monofluorotrichloromethane, difluorodichloromethane, tetrafluorodichloroethane and 1,1-difluoroethane sold in particular under the trade name Dymel 152 A® by the company DuPont.

Use may also be made, as propellant, of carbon dioxide gas, nitrous oxide, nitrogen or compressed air.

Preferably, the antiperspirant cosmetic composition according to the invention comprises a propellant chosen from volatile hydrocarbons.

More preferentially, the propellant is chosen from isopropane, n-butane, isobutane, pentane and isopentane, and mixtures thereof.

The weight ratio between the liquid phase and the propellant gas varies in a ratio from 5/95 to 50/50, preferably from 10/90 to 40/60 and more preferentially from 15/85 to 30/70.

Preferably, the composition according to the invention comprises:
- an oily phase comprising one or more volatile oils A chosen from volatile hydrocarbon-based oils, and preferably C₈-C₁₆ isoalkanes and more particularly isododecane; and
- one or more non-volatile hydrocarbon-based oils, preferably chosen from fatty acid esters, and more particularly isopropyl palmitate, and
- one or more antiperspirant active agents chosen from aluminium salts and more particularly aluminium chlorohydrate and/or aluminium sesquichlorohydrate, and
- a dispersion of particles of methyl acrylate/ethyl acrylate (50/50 by weight) copolymer, stabilized by a random copolymer stabilizer containing 75.2% by weight of isobornyl acrylate, 3% of methyl acrylate, 3% of ethyl acrylate and 18.8% of monomethacryloyloxypropyl polydimethylsiloxane, in isododecane and
- one or more propellants.

The invention also relates to a cosmetic process for treating human perspiration, and optionally body odours associated with human perspiration, which consists in applying to the surface of the skin an effective amount of the cosmetic composition as described previously.

The application time of the cosmetic composition on the surface of the skin may range from 0.5 to 10 seconds and preferably from 1 to 5 seconds.

The cosmetic composition in accordance with the invention may be applied several times to the surface of the skin.

In particular, the cosmetic treatment process according to the invention consists in applying to the surface of the armpits an effective amount of the cosmetic composition as described above.
The invention also relates to the use of said composition for the cosmetic treatment of human perspiration.

Another subject of the present invention is an aerosol device constituted of a container comprising an aerosol composition as defined previously and of a means for dispensing said composition.

The dispensing means, which forms a part of the aerosol device, is generally constituted of a dispensing valve controlled by a dispensing head, which itself comprises a nozzle via which the aerosol composition is vaporized. The container containing the pressurized composition may be opaque or transparent. It may be made of glass, of polymer or of metal, optionally coated with a protective varnish layer.

The examples that follow illustrate the present invention. The amounts of the ingredients are expressed as weight percentages relative to the total weight of the composition.

### Method for measuring the transfer

The measurement of the transfer onto clothing was performed according to the protocol described below:
Each of the compositions to be studied was deposited on an imitation leather article sold under the name Supplale® by the company Idemitsu Technofine, which is bonded onto a rigid support. This deposition is performed by spraying the aerosol for 2 seconds at a distance of 15 cm from the support.

After 24 h, a black cotton fabric, which is dry or has been sprayed with artificial* sweat, is deposited on the imitation leather article. A pressure of 1 Newton is applied with a rotational motion over 1 rotation at a speed of 3.14 cm/sec.

The fabric is scanned with a scanner sold under the name Epson V500 Scanner (16-bits grey setting, resolution 300 dpi).

The level of grey of the scans is then analyzed using image software J which has a grey level ranging from 0 to 255. The higher the grey level value, the stronger the marks. It is thus sought to obtain the lowest possible grey level values.

The transfer evaluation is also performed by observation of the residual deposit on the synthetic leather plate:
The persistence is considered as being very good when the deposit is unchanged after the fabric has been passed over.

It is considered as being good when the deposit is visible after the fabric has been passed over.
It is considered as being poor when the deposit is no longer (or only slightly) visible after the fabric has been passed over.

***Composition of the artificial sweat:**

| Ingredients | Weight% |
|---|---|
| NaCl | 0.5% (85.6 mM) |
| lactic acid | 0.1% (11.1 mM) |
| Urea | 0.1% (14.7 mM) |
| Albumin | 0.10% |
| NH₄OH | qs pH 6.5 |

### Example 1

In a first step, 120 g of isododecane, 50 g of isobornyl acrylate, 2 g of methyl acrylate, 2 g of methyl acrylate, 12.5 g of monomethacryloyloxypropyl polydimethylsiloxane (X-22-2426 from Shin Etsu) and 0.665 g of tert-butyl peroxy-2-ethylhexanoate (Trigonox 21S from Akzo) are introduced into a reactor.

The isobornyl acrylate / methyl acrylate / ethyl acrylate / monomethacryloyloxypropyl polydimethylsiloxane weight ratio is 75.2/3/3/18.8.

The mixture was heated to 90°C under argon with stirring.

After 2 hours of reaction, 78 g of isododecane were added to the reactor feedstock and the mixture was heated to 90°C.

In a second step, a mixture of 91.5 g of methyl acrylate, 91.5 g of ethyl acrylate, 183 g of isododecane and 1.83 g of Trigonox 21S were run in over the course of one hour, and the mixture was left to react for 7 hours. 0.3 litre of isododecane were then added and part of the isododecane was evaporated off to obtain a solids content of 45% by weight.

A dispersion of particles of methyl acrylate/ethyl acrylate (50/50 by weight) copolymer, stabilized by a random copolymer stabilizer containing 75.2% by weight of isobornyl acrylate, 3% of methyl acrylate, 3% of ethyl acrylate and 18.8% of monomethacryloyloxypropyl polydimethylsiloxane, in isododecane, was obtained.

The oily dispersion contains in total (stabilizer + particles) 20% of isobornyl acrylate, 37.5% of methyl acrylate, 37.5% of ethyl acrylate and 5% of monomethacryloyloxypropyl polydimethylsiloxane.

The particles of the polymer of the dispersion have a number-average size of between about 170 nm and 200 nm.

### Example 2

The formulae tested in aerosol form comprise a base manufactured according to the process described below and containing the ingredients mentioned in the following table:

| **Phase** | **Ingredients** | **Invention Preparation Example 2** | **Comparative Preparation C2** |
|---|---|---|---|
| A | Isopropyl palmitate⁽¹⁾ | 7.24 | 23.88 |
| | Isododecane⁽²⁾ | qs 100 | qs 100 |
| | (Methyl Acrylate)-co-(Ethyl Acrylate)-co-(Isobornyl Acrylate)-co-(MPDMS 12k) (37.5/37.5/20/5) Copolymer of Example 1 | 38.8 | 0 |
| B | Disteardimonium hectorite ⁽³⁾ | 0.86 | 0.86 |
| C | Propylene carbonate⁽⁴⁾ | 0.26 | 0.26 |
| D | Aluminium chlorohydrate ⁽⁵⁾ | 35 | 35 |

| | | | |
|---|---|---|---|
| ⁽¹⁾ sold under the trade name Isopropyl palmitate by the company BASF (Cognis) ⁽²⁾ sold under the trade name Isododecane by the company Ineos ⁽³⁾ sold under the trade name Bentone 38VCG by the company Elementis ⁽⁴⁾ sold under the trade name Jeffsol propylene carbonate by the company Huntsman ⁽⁵⁾ sold under the trade name Reach 103 by the company Summit Reheis | | | |

Phase A was mixed with stirring. Phase (B) was introduced slowly into phase (A) and the mixture was then left to swell for five minutes. (C) was introduced. The mixture was stirred vigorously until good homogenization was obtained. The aluminium chlorohydrate was then added portionwise. Stirring was continued to obtain good homogenization.

The bases thus formulated were packaged in cans and a propellant was added to the above preparations according to the following schemes:

| **Aerosol composition** | **Invention Example 2** | **Comparative C2** |
|---|---|---|
| **Preparation Ex 2** | 15 | - |
| **Preparation C2** | - | 15 |
| **Isobutane** | 85 | 85 |

### Results regarding the transfer-resistance efficacy

The Example 2 aerosol, with an oily dispersion, was sprayed under the conditions described above and the results obtained in comparison with the aerosol without oily dispersion (comparative C2) are described in the table below:

| **Aerosol** | **Example 2 (invention)** | **Comparative Example C2 without Oily Dispersion** |
|---|---|---|
| **Level of grey dry** | 50.5 ± 1.2 | 69.2 ± 1.2 |
| **Level of grey with the artificial sweat** | 47.2 ± 0.3 | 47.2 ± 0.3 |

It is noted that, in the case of the composition of Example 2, the persistence was better than that of composition C2.

## Claims

1. Anhydrous composition in aerosol form comprising, in particular in a physiologically acceptable medium:
a) an oily phase comprising at least one volatile oil A, and
b) at least one antiperspirant active agent chosen from aluminium and/or zirconium salts or complexes, and
c) at least one dispersion of particles of methyl acrylate/ethyl acrylate (50/50 by weight) copolymer, stabilized by a random copolymer stabilizer containing 75.2% by weight of isobornyl acrylate, 3% of methyl acrylate, 3% of ethyl acrylate and 18.8% of monomethacryloyloxypropyl polydimethylsiloxane, in isododecane, and
d) at least one propellant;
wherein the liquid phase of said composition comprises a water content of less than 5% by weight, relative to the weight of the composition.

2. Composition according to one of the preceding claims, **characterized in that** the polymer of the particles is present in a content ranging from 20% to 60% by weight, relative to the total weight of the dispersion.

3. Composition according to one of the preceding claims, **characterized in that** the polymer particles have an average size ranging from 50 to 500 nm, in particular ranging from 75 to 400 nm and better still ranging from 100 to 250 nm.

4. Composition according to one of the preceding claims, **characterized in that** the antiperspirant active agent is aluminium chlorohydrate and/or aluminium sesquichlorohydrate.

5. Composition according to one of the preceding claims, **characterized in that** the propellant is chosen from dimethyl ether, volatile hydrocarbons such as n-butane, propane, isopropane, isobutane, pentane and isopentane, and mixtures thereof, optionally with at least one chlorohydrocarbon and/or fluorohydrocarbon.

6. Composition according to one of the preceding claims, **characterized in that** the volatile oil A is chosen from C₈-C₁₆ volatile hydrocarbon-based oils, preferably from C₈-C₁₆ isoalkanes and linear C₈-C₁₆ alkanes, and mixtures thereof, and more particularly isododecane.

7. Composition according to any one of the preceding claims, **characterized in that** the oily phase comprises, in addition, at least one non-volatile oil, preferably chosen from non-volatile hydrocarbon-based oils, more preferentially chosen from hydrogenated polyisobutene oils, ethers, fatty acid esters, fatty alcohols and mixtures thereof, and more particularly fatty acid esters and even more particularly isopropyl palmitate.

8. Composition according to any one of the preceding claims, **characterized in that** it comprises:
- an oily phase comprising at least one volatile oil A chosen from volatile hydrocarbon-based oils, and preferably C₈-C₁₆ isoalkanes and more particularly isododecane; and
- at least one non-volatile hydrocarbon-based oil B, preferably chosen from fatty acid esters, and more particularly isopropyl palmitate, and
- at least one antiperspirant active agent chosen from aluminium salts and more particularly aluminium chlorohydrate and/or aluminium sesquichlorohydrate, and
- a dispersion of particles of methyl acrylate/ethyl acrylate (50/50 by weight) copolymer, stabilized by a random copolymer stabilizer containing 75.2% by weight of isobornyl acrylate, 3% of methyl acrylate, 3% of ethyl acrylate and 18.8% of monomethacryloyloxypropyl polydimethylsiloxane, in isododecane.

9. Cosmetic process for treating human perspiration, and optionally body odours associated with human perspiration, which consists in applying to the surface of the skin an effective amount of the composition as defined in any one of Claims 1 to 8.

10. Aerosol device constituted of a container comprising an aerosol composition as defined according to any one of Claims 1 to 8 and of a means for dispensing said aerosol composition.

## Patentansprüche

1. Wasserfreie Zusammensetzung in Aerosolform, umfassend, insbesondere in einem physiologisch unbedenklichen Medium:
a) eine ölige Phase, die mindestens ein flüchtiges Öl A umfasst, und
b) mindestens einen Antiperspirant-Wirkstoff, der aus Aluminium- und/oder Zirkoniumsalzen oder -komplexen ausgewählt ist, und
c) mindestens eine Dispersion von Teilchen von Methylacrylat/Ethylacrylat-Copolymer (Gewichtsverhältnis 50/50), die durch ein statistisches Copolymer als Stabilisator, das 75,2 Gew.-% Isobornylacrylat, 3 % Methylacrylat, 3 % Ethylacrylat und 18,8 % Monomethacryloyloxypropylpolydimethylsiloxan enthält, stabilisiert sind, in Isododecan und
d) mindestens ein Treibmittel;
wobei die flüssige Phase der Zusammensetzung einen Wassergehalt von weniger als 5 Gew.-%, bezogen auf das Gewicht der Zusammensetzung, umfasst.

2. Zusammensetzung nach einem der vorhergehenden Ansprüche, **dadurch gekennzeichnet, dass** das Polymer der Teilchen in einem Gehalt im Bereich von 20 bis 60 Gew.-%, bezogen auf das Gesamtgewicht der Dispersion, vorliegt.

3. Zusammensetzung nach einem der vorhergehenden Ansprüche, **dadurch gekennzeichnet, dass** die Polymerteilchen eine mittlere Größe im Bereich von 50 bis 500 nm, insbesondere im Bereich von 75 bis 400 nm und noch besser im Bereich von 100 bis 250 nm aufweisen.

4. Zusammensetzung nach einem der vorhergehenden Ansprüche, **dadurch gekennzeichnet, dass** es sich bei dem Antiperspirant-Wirkstoff um Aluminiumchlorhydrat und/oder Aluminiumsesquichlorhydrat handelt.

5. Zusammensetzung nach einem der vorhergehenden Ansprüche, **dadurch gekennzeichnet, dass** das Treibmittel aus Dimethylether, flüchtigen Kohlenwasserstoffen wie n-Butan, Propan, Isopropan, Isobutan, Pentan und Isopentan und Mischungen davon, gegebenenfalls mit einem Chlorkohlenwasserstoff und/oder Fluorkohlenwasserstoff, ausgewählt ist.

6. Zusammensetzung nach einem der vorhergehenden Ansprüche, **dadurch gekennzeichnet, dass** das flüchtige Öl A aus flüchtigen Ölen auf Basis von C₈-C₁₆-Kohlenwasserstoffen, vorzugsweise aus C₈-C₁₆-Isoalkanen und linearen C₈-C₁₆-Alkanen, und Mischungen davon und spezieller Isododecan ausgewählt ist.

7. Zusammensetzung nach einem der vorhergehenden Ansprüche, **dadurch gekennzeichnet, dass** die ölige Phase außerdem mindestens ein nichtflüchtiges Öl umfasst, das vorzugsweise aus nichtflüchtigen Ölen auf Kohlenwasserstoffbasis ausgewählt ist, weiter bevorzugt aus hydrierten Polyisobutylenölen, Ethern, Fettsäureestern, Fettalkoholen und Mischungen davon und spezieller Fettsäureestern und noch spezieller Isopropylpalmitat ausgewählt ist.

8. Zusammensetzung nach einem der vorhergehenden Ansprüche, **dadurch gekennzeichnet, dass** sie Folgendes umfasst:
- eine ölige Phase, die mindestens ein flüchtiges Öl A, das aus flüchtigen Ölen auf Kohlenwasserstoffbasis, vorzugsweise aus C₈-C₁₆-Isoalkanen und spezieller Isododecan ausgewählt ist, umfasst, und
- mindestens ein nichtflüchtiges Öl B auf Kohlenwasserstoffbasis, das vorzugsweise aus Fettsäureestern und spezieller Isopropylpalmitat ausgewählt ist und
- mindestens einen Antiperspirant-Wirkstoff, der aus Aluminiumssalzen und spezieller Aluminiumchlorhydrat und/oder Aluminiumsesquichlorhydrat ausgewählt ist, und
- eine Dispersion von Teilchen von Methylacrylat/Ethylacrylat-Copolymer (Gewichtsverhältnis 50/50), die durch ein statistisches Copolymer als Stabilisator, das 75,2 Gew.-% Isobornylacrylat, 3 % Methylacrylat, 3 % Ethylacrylat und 18,8 % Monomethacryloyloxypropylpolydimethylsiloxan enthält, stabilisiert sind, in Isododecan.

9. Kosmetisches Verfahren zum Behandeln von menschlicher Perspiration und gegebenenfalls mit menschlicher Pespiration verbundenen Körpergerüchen, das darin besteht, dass man auf die Oberfläche der Haut eine wirksame Menge der Zusammensetzung gemäß einem der Ansprüche 1 bis 8 aufbringt.

10. Aerosolvorrichtung, bestehend aus einem Behälter, der eine Aerosolzusammensetzung nach einem der Ansprüche 1 bis 8 umfasst, und einem Mittel zur Abgabe der Aerosolzusammensetzung.

## Revendications

1. Composition anhydre sous forme d'aérosol comprenant, en particulier dans un milieu physiologiquement acceptable :
a) une phase huileuse comprenant au moins une huile volatile A et
b) au moins un principe actif anti-transpirant choisi parmi les sels ou complexes d'aluminium et/ou de zirconium et
c) au moins une dispersion de particules de copolymère d'acrylate de méthyle/acrylate d'éthyle (50/50 en poids), stabilisée par un stabilisant copolymère statistique contenant 75,2 % en poids d'acrylate d'isobornyle, 3 % d'acrylate de méthyle, 3 % d'acrylate d'éthyle et 18,8 % de monométhacryloyloxypropylpolydiméthylsiloxane, dans de l'isododécane et
d) au moins un agent propulseur ;
la phase liquide de ladite composition comprenant une teneur en eau inférieure à 5 % en poids, par rapport au poids de la composition.

2. Composition selon l'une des revendications précédentes, **caractérisée en ce que** le polymère des particules est présent en une teneur allant de 20 % à 60 % en poids, par rapport au poids total de la dispersion.

3. Composition selon l'une des revendications précédentes, **caractérisée en ce que** les particules de polymère ont une taille moyenne allant de 50 à 500 nm, en particulier allant de 75 à 400 nm et mieux encore allant de 100 à 250 nm.

4. Composition selon l'une des revendications précédentes, **caractérisée en ce que** le principe actif anti-transpirant est le chlorohydrate d'aluminium et/ou le sesquichlorohydrate d'aluminium.

5. Composition selon l'une des revendications précédentes, **caractérisée en ce que** l'agent propulseur est choisi parmi l'oxyde de diméthyle, les hydrocarbures volatils tels que le n-butane, le propane, l'isopropane, l'isobutane, le pentane et l'isopentane et les mélanges de ceux-ci, éventuellement avec au moins un hydrocarbure chloré et/ou un hydrocarbure fluoré.

6. Composition selon l'une des revendications précédentes, **caractérisée en ce que** l'huile volatile A est choisie parmi les huiles à base d'hydrocarbures volatils en C₈-C₁₆, de préférence parmi les isoalcanes en C₈-C₁₆ et les alcanes en C₈-C₁₆ linéaires, et les mélanges de ceux-ci et plus particulièrement l'isododécane.

7. Composition selon l'une quelconque des revendications précédentes, **caractérisée en ce que** la phase huileuse comprend, de plus, au moins une huile non volatile, de préférence choisie parmi les huiles à base d'hydrocarbures non volatils, plus préférablement choisie parmi les huiles de polyisobutène hydrogéné, les éthers, les esters d'acides gras, les alcools gras et les mélanges de ceux-ci et plus particulièrement les esters d'acides gras et encore plus particulièrement le palmitate d'isopropyle.

8. Composition selon l'une quelconque des revendications précédentes, **caractérisée en ce qu'**elle comprend :
- une phase huileuse comprenant au moins une huile volatile A choisie parmi les huiles à base d'hydrocarbures volatils et de préférence les isoalcanes en C₈-C₁₆ et plus particulièrement l'isododécane ; et
- au moins une huile à base d'hydrocarbures non volatils B, de préférence choisie parmi les esters d'acides gras et plus particulièrement le palmitate d'isopropyle et
- au moins un principe actif anti-transpirant choisi parmi les sels d'aluminium et plus particulièrement le chlorohydrate d'aluminium et/ou le sesquichlorohydrate d'aluminium et
- une dispersion de particules de copolymère d'acrylate de méthyle/acrylate d'éthyle (50/50 en poids), stabilisée par un stabilisant copolymère statistique contenant 75,2 % en poids d'acrylate d'isobornyle, 3 % d'acrylate de méthyle, 3 % d'acrylate d'éthyle et 18,8 % de monométhacryloyloxypropylpolydiméthylsiloxane, dans de l'isododécane.

9. Procédé cosmétique pour le traitement de la transpiration humaine, et éventuellement d'odeurs corporelles associées à la transpiration humaine, qui consiste à appliquer sur la surface de la peau une quantité efficace de la composition telle que définie dans l'une quelconque des revendications 1 à 8.

10. Dispositif aérosol constitué d'un récipient comprenant une composition d'aérosol telle que définie selon l'une quelconque des revendications 1 à 8 et un moyen pour la distribution de ladite composition d'aérosol.
